(19) Europäisches Patentamt
European Patent Office
Office européen des brevets

(11) Publication number: 0 018 594 B1

(12) EUROPEAN PATENT SPECIFICATION

(45) Date of publication of patent specification: 21.08.85

(21) Application number: 80102202.1

(22) Date of filing: 24.04.80

(51) Int. Cl.⁴: C 07 D 205/08, C 07 D 409/06, C 07 D 498/04, C 07 F 7/10 // (C07D498/04, 265:00, 205:00)

# (54) Process for the preparation of 3-(1-hydroxyethyl)-azetidinones.

(30) Priority: 27.04.79 US 34053

(43) Date of publication of application: 12.11.80 Bulletin 80/23

(45) Publication of the grant of the patent: 21.08.85 Bulletin 85/34

(84) Designated Contracting States: AT BE CH DE FR GB IT LI LU NL SE

(56) References cited:
EP-A-0 007 973
EP-A-0 017 992
EP-A-0 026 817
EP-A-0 030 031
EP-A-0 030 032
EP-A-0 037 080
EP-A-0 037 082
DE-A-2 751 597
DE-A-2 751 624

JOURNAL OF THE AMERICAN CHEMICAL SOCIETY, Vol. 100, No. 1, 4 January 1978 D.B.R. JOHNSTON et al. "Total Synthesis of (+,-)-Thienamycin" pages 313 to 315

(73) Proprietor: MERCK & CO. INC.
126, East Lincoln Avenue P.O. Box 2000
Rahway New Jersey 07065 (US)

(72) Inventor: Bouffard, Aileen F.
1521 Cooper Road
Scotch Plains New Jersey 07076 (US)
Inventor: Christensen, Burton G.
195 Watchung Terrace
Scotch Plains New Jersey 07076 (US)
Inventor: Ratcliffe, Ronald W.
234 Matawan Avenue
Matawan New Jersey 07747 (US)
Inventor: Salzmann, Thomas N.
387 Brook Avenue
North Plainfield New Jersey 07062 (US)

(74) Representative: Abitz, Walter, Dr.-Ing. et al
Abitz, Morf, Gritschneder P.O. Box 86 01 09
D-8000 München 86 (DE)

EP 0 018 594 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

Background of the Invention

This invention relates to a stereocontrolled process for the preparation of certain 3-(1-hydroxyethyl)-azetidinones 4 which are useful in the synthesis of the antibiotic thienamycin and antibiotic, side chain analogues thereof. The process may be summarized as follows:

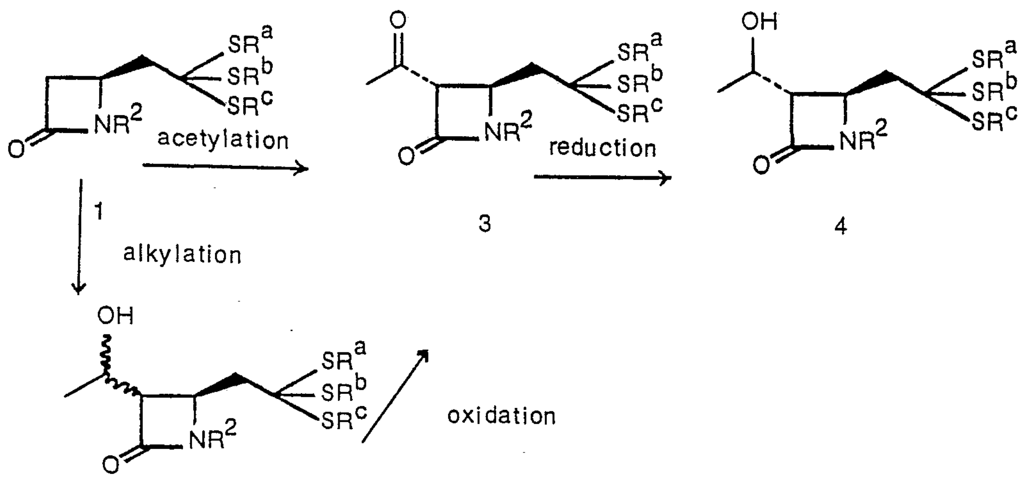


1 3 4

2

DIAGRAM I

Relative to the above scheme, $R^a$, $R^b$ and $R^c$ are independently chosen from alkyl having 1—6 carbon atoms, phenylalkyl and phenyl. Typically, $R^a$, $R^b$ and $R^c$ are chosen from groups such as methyl, ethyl, benzyl, ethoxybenzyl, trityl, isopropyl, t-butyl, and phenyl, for example. Further, any pair of $R^a$, $R^b$ and $R^c$ may be joined together to form the radical $—(CH_2)_3—$ to bridge two sulphur atoms.

The precise identity of protecting group $R^2$ is not critical, provided only that the group does not interfere with the indicated course of reaction. However, triorganosilyl groups and groups such as 2-tetrahydropyranyl are preferred. Representative of such silyl protecting groups are those wherein the organo moiety is chosen from phenyl and alkyl having 1—6 carbon atoms, such as trimethylsilyl, t-butyldimethylsilyl, triphenylsilyl, for example.

The final product 4 of the process of the present invention is encountered in various schemes for the total synthesis of thienamycin. Some of these schemes, which demonstrate the utility of 4, are published; others are recited in European Patent Application Publication 007973 (application 79101307.1, filed 1 May 1979).

Similar processes leading to products with different substitution are described in European Patent Applications Nos. 0017992,0026817, 0030031, 0030032, 0037080 and 0037082. These applications are to be considered under Article 57 (3) (4) EPC.

Detailed Description of the Invention

Relative to Diagram I, above, the alkylation reaction 1 → 2 is accomplished by treating 1 in a solvent such as tetrahydrofuran, dimethoxyethane, diethylether, hexamethylphosphoramide, glyme, dimethylforamide, dimethylsulfoxide, toluene, N,N,N',N'-tetramethylethylene diamine, or the like at a temperature of from −100 to 0°C with a strong base such as lithium diisopropylamide, lithium hexamethyldisilizide, mesitylithium, lithium-2,2,6,6-tetramethylpiperidide, potassium hydride, sodium amide, t-butyl lithium, s-butyl lithium, potassium triphenylmethide, or potassium hexamethyldisilizide followed by the addition of an equivalent to 10-fold excess of acetaldehyde. This reaction gives a mixture of isomers from which the desired *trans*-R form (4) can be conveniently separated by chromatography or crystallization. In the alternative, 2 can be oxidized to 3 followed by reduction to 4.

The oxidation 2 → 3 is accomplished with an oxidizing agent; representative of such non-critical oxidizing agents are: dipyridinium chromium (VI) oxide, trifluoroacetic anhydride-dimethylsulfoxide-triethylamine, pyridinium dichromate, aceticanhydride-dimethylsulfoxide, oxalyl chloride-dimethylsulfoxide-triethylamine, N-chlorosuccinimide-dimethylsulfide, Amberlyst A®—25/$HCrO_4^-$, chromium trioxide-3,5-dimethylpyrazole, methanesulfonic anhydride-dimethylsulfoxide-triethylamine, pyridinium chlorochromate, pyridinium dichromate, dicyclohexyldiimide-dimethylsulfoxide, in a solvent such as methylene chloride, acetonitrile, hexamethylphosphoramide, toluene, dimethylformamide or dimethylsulfoxide at a temperature of from −78 to 25°C for from 5 minutes to 5 hours.

The reduction 3 → 4 is accomplished by contacting 3 with a reducing agent such as potassium tri(sec-butyl)borohydride, lithium tri(sec-butyl)borohydride, sodium borohydride, sodium tris(methoxyethoxy)aluminumhydride, zinc borohydride, potassium borohydride, lithium triethylborohydride, lithium tri-*n*-butylborohydride, lithium trisiamylborohydride, trimethyl-*tert*-butylaminomagnesium hydride, 2,6-diisopropylphenoxymagnesium hydride or lithium tri-t-butoxy aluminum hydride in a solvent such as diethylether, tetrahydrofuran, toluene, dimethylforamide, hexamethylphosphoramide, 2-propanol, ethanol, pentane, or mixtures of any of the above at a temperature of from −78° to 25°C. The reaction can conveniently be conducted in the presence of an added complexing salt such as potassium iodide or magnesium bromide. Lithium tri(sec-butyl)borohydride is preferred since it yields 4 in predominantly the desired *trans*-R form. It should be noted that the desired *trans*-R form can be separated from contaminating *trans*-S in the 4 mixture by crystallization or chromatography. Further, it should be noted that the above described oxidation-reduction scheme can be repeated on mixed products 4 to enhance concentration of *trans*-R.

The acetylation 1 → 3 is accomplished by treating 1 with two or more equivalents of a base such as lithium diisopropylamide, lithium hexamethyldisilizide, mesitylithium or lithium 2,2,6,6-tetramethylpyridide in a solvent such as tetrahydrofuran, diethylether or dimethoxyethane at a temperature of from −100 to −20°C with an acetylating agent such as N-acetyl imidazole and other activated acylating agents such as thiol esters of acetic acid:

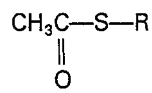

wherein R is alkyl having from 1—6 carbon atoms, phenyl and phenylalkyl; acetic acid halides such as

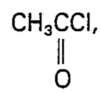

and

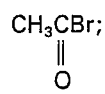

acetic anhydride; and mixed anhydrides such as:

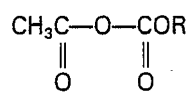

wherein R is alkyl having 1—6 carbon atoms. Addition of the 1 plus base mixture to the acylating agent is preferred.

In the foregoing word description of the process of the present invention, it is to be understood that there is considerable latitude in selection of precise reaction parameters. Suggestion of this latitude and its breadth is generally indicated by the enumeration of equivalent solvent systems, temperature ranges, protecting groups, and range of identities of involved reagents. Further, it is to be understood that the presentation of the synthetic scheme as comprising distinct steps in a given sequence is more in the nature of a descriptive convenience than as a necessary requirement; for one will recognize that the mechanically dissected scheme represents a unified scheme of synthesis and that certain steps, in actual practice, are capable of being merged, conducted simultaneously, or effected in a reverse sequence without materially altering the progress of synthesis.

The following examples are included for a further understanding of the invention.

Example 1

Preparation of (3S,4R)-1-(t-butyldimethylsilyl)-3-[(R)-1-hydroxyethyl]-4-[2,2,2-tri(methylthio)ethyl]azetidin-2-one

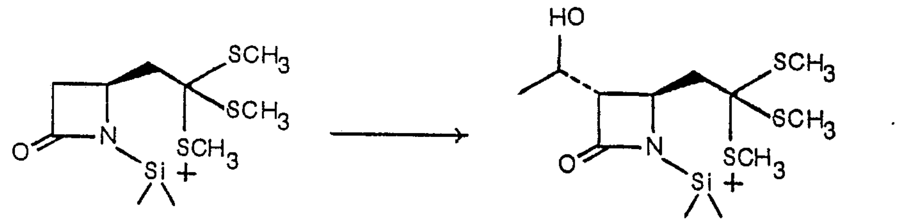

n-Butyllithium (14.8 ml of 2.5N hexane solution, 37.0 mmol) is added by syringe to a solution of diisopropylamine (3.74 g, 37.0 mmol) in 180 ml of freshly distilled tetrahydrofuran at −78°C. The resulting solution is stirred at −78°C for 15 min. prior to the addition of a solution of (4S)-1-(t-butyldimethylsilyl)-4-[2,2,2-tri(methylthio)ethyl]azetidin-2-one (12.34 g, 35.16 mmol) in 35 ml of tetrahydrofuran. This solution is stirred at −78°C for 10 min. prior to the addition of acetaldehyde (4.62 g, 105 mmol). The solution is stirred for an additional 5 min. at −78°C and then quenched by addition of saturated aqueous ammonium chloride solution, and allowed to warm to room temperature. The mixture is poured into 250 ml of ether and washed with water (2 × 100 ml) and brine and dried over magnesium sulfate. Removal of solvents *in vacuo* gives an oil which is chromatographed on a silica gel column (1:1 ether:petroleum ether) to give (3*S*,4R)-1-(t-butyldimethylsilyl)-3-[(R)-1-hydroxyethyl]-4-[2,2,2-tri(methylthio)ethyl]azetidin-2-one (7.0 g, 50.4%) at $R_f = 0.2$. The product can be recrystallized from petroleum ether. Alternatively, the trans R product can be isolated from the crude reaction mixture by direct crystallization from a petroleum ether solution.

Example 2

Preparation of (3S,4R)-1-(t-Butyldimethylsilyl)-3-(1-oxoethyl)-4-[2,2,2-tri(methylthio)ethyl]azetidin-2-one

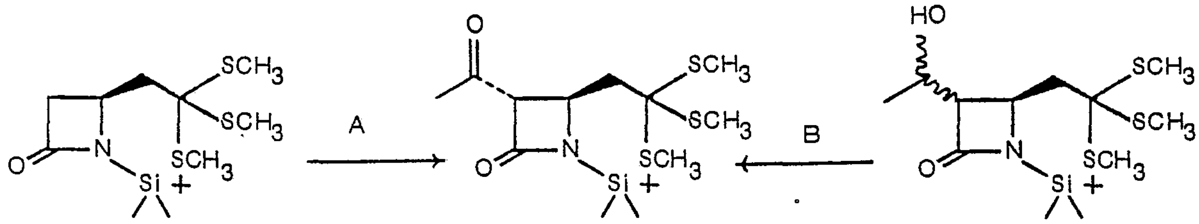


*A.* n-Butyllithium (2.43 ml of 2.4m solution, 5.84 mmol) is added by syringe to a solution of diisopropylamine (591 mg, 5.84 mmol) in 25 ml of freshly distilled tetrahydrofuran at −78°C. The resulting solution is stirred at −78°C for 15 minutes prior to the addition of a solution of (4*R*)-1-(t-butyldimethylsilyl)-4-[2,2,2-tri(methylthio)ethyl]azetidin-2-one (1.00 g, 2,85 mmol) in tetrahydrofuran (5 ml). This solution is stirred at −78°C for 15 minutes, then added *via* a Teflon tube to a mixture of N-acetylimidazole (642 mg, 5.84 mmol) in 25 ml of THF at −78°C. The resulting yellow reaction mixture is stirred at −78°C for 10 minutes, then quenched by addition of saturated aqueous ammonium chloride solution. The mixture is diluted with ether (200 ml) and washed with 2.5N hydrochloric acid solution (50 ml), water (50 ml) and brine (50 ml) and dried over magnesium sulfate. Removal of solvents *in vacuo* gives a yellow oil which is chromatographed on silica gel (30% ether in petroleum ether) to yield (3*S*,4*R*)-1-(t-butyldimethylsilyl)-3-(1-oxoethyl)-4-[2,2,2-tri(methylthio)ethyl]azetidin-2-one. n.m.r. ($CDCl_3$) δ 4.42 (m, 1), δ 4.32 (d, 1) δ 2.35 (m, 2), δ 2.32 (s, 3), δ 2.2 (s, 9), δ 0.98 (s, 9), δ 0.3 (2s, 6).

*B.* Trifluoroacetic anhydride (400 mg., 1.905 mmol) is added by syringe to a solution of dimethyl sulfoxide (2.53 mmol) in dry methylene chloride (5 ml) at −78°C. The resulting mixture is stirred at −78°C for 30 minutes prior to the addition of a solution of (3RS,4R)-1-(t-butyldimethylsilyl)-3-[(RS)-1-hydroxyethyl]-4-[2,2,2-tri(methylthio)ethyl]azetidin-2-one (500 mg., 1.27 mmol) in dry $CH_2Cl_2$ (1 ml). The resulting solution is stirred for 30 minutes prior to the addition of triethylamine (360 mg., 3.56 mmol). The cooling bath is removed. After 40 minutes, the reaction mixture is diluted with $CH_2Cl_2$, washed with water and brine and dried over magnesium sulfate. Removal of solvents *in vacuo* gives an oil which is purified as above. Yield 432 mg (86%).

Example 3

Preparation of (3*S*,4*R*)-1-(t-butyldimethylsilyl)-3-[(*R*)-1-hydroxyethyl]-4-[2,2,2-tri(methylthio)ethyl]azetidin-2-one

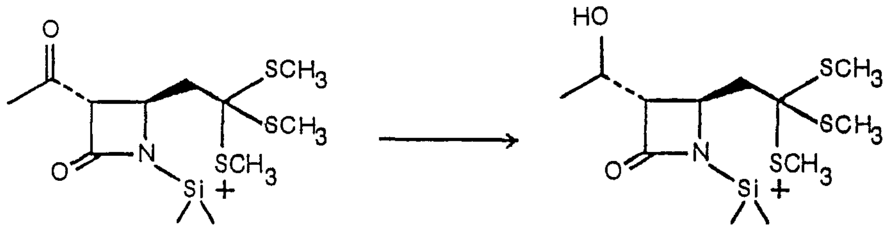

K-Selectride® (3.64 ml of 0.5M, 1.82 mmol) is added by syringe to a mixture of potassium iodide (126 mg, 0.758 mmol and (3*S*,4*R*)-1-(t-butyldimethylsilyl)-3-(1-oxoethyl)-4-[2,2,2-tri(methylthio)ethyl]azetidin-2-one (298 mg, 0.758 mmol in freshly distilled ether (8 ml) at room temperature. The resulting mixture is stirred at room temperature for 2.5 hours, then quenched by the addition of acetic acid (218 mg, 3.64 mmol). The resulting mixture is diluted with ethyl acetate (25 ml) and filtered through celite. Removal of solvents *in vacuo* gives an oil which is chromatographed on silica gel (ether:petroleum ether) to yield 252m of (3*S*,4*R*)-1-(t-butyldimethylsilyl)-3-[(*R*)-1-hydroxyethyl]-4-[2,2,2-tri(methylthio)ethyl]azetidin-2-one. N.M.R. (*R* isomer, $CDCl_3 + D_2O$) δ 4.15 (dq, 1), δ 3.95 (ddd, 1, J = 9.5, 2.3), δ 3.26 (dd, 1, J = 8, 2.3), δ 2.37 (m, 2), δ 2.16 (s, 9), δ 1.37 (d, 3, J = 6, 6), δ 1.0 (s, 9), δ 0.26 (s, 6).

**Claims**

1. A process for preparing:

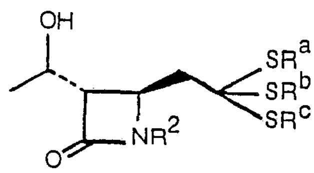

comprising treating:

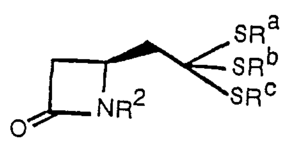

with an acetylating agent to yield:

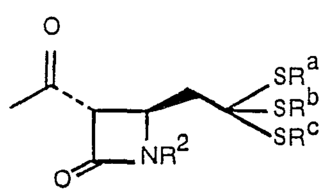

followed by treating with a reducing agent; wherein $R^2$ is selected from hydrogen or a protecting group; $R^a$, $R^b$ and $R^c$ are independently selected from: alkyl, phenyl and phenylalkyl, wherein the alkyl has 1—6 carbon atoms, and any single pair of $R^a$, $R^b$ or $R^c$ may be joined to form $—(CH_2)_3—$.

2. A process according to Claim 1 wherein $R^2$ is hydrogen or a triorganosilyl protecting group; $R^dR^eR^fSi$- wherein $R^d$, $R^e$ and $R^f$ are independently selected from alkyl having 1—6 carbon atoms; and wherein $R^a$, $R^b$ and $R^c$ are independently selected from the group consisting of alkyl having 1—6 carbon atoms, phenyl and alkylphenyl having 7—12 carbon atoms.

3. A process according to Claim 1 wherein the acetylating agent is N-acetyl imidazole.

4. A process according to Claims 2 or 3 wherein $R^2$ is triorganosilyl.

5. A process for preparing:

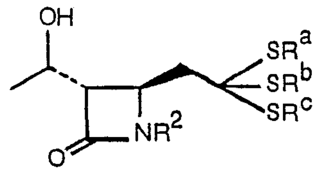

comprising treating:

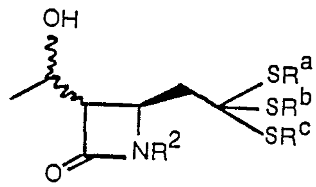

with an oxidizing agent to yield:

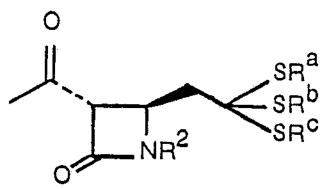

followed by treating with a reducing agent; wherein $R^2$, $R^a$, $R^b$ and $R^c$ are defined as in claim 1.

6. A process according to Claim 5 wherein $R^2$ is hydrogen or a triorganosilyl protecting group: $R^dR^eR^fSi$- wherein $R^d$, $R^e$ and $R^f$ are independently selected from alkyl having 1—6 carbon atoms; and wherein $R^a$, $R^b$ and $R^c$ are independently selected from the group consisting of alkyl having 1—6 carbon atoms, phenyl and alkylphenyl having 7—12 carbon atoms.

7. A process according to Claims 5 or 6 wherein $R^2$ is triorganosilyl.

**Revendications**

1. Un procédé pour préparer:

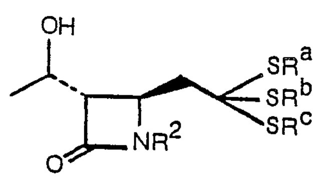

comprenant le traitement de:

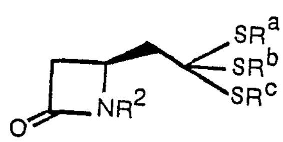

avec un agent d'acétylation pour donner:

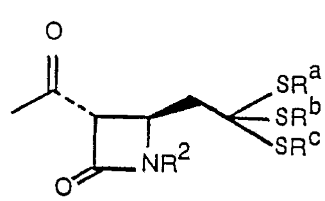

suivi du traitement avec un agent réducteur; dans lequel $R^2$ est choisi parmi l'hydrogène ou un groupe

protecteur; $R^a$, $R^b$ et $R^c$ sont choisis indépendamment parmi: les groupes alkyle, phényle et phénylalkyle, dans lesquels la fraction alkyle possède 1 à 6 atomes de carbone et toute paire unique parmi $R^a$, $R^b$ ou $R^c$ peut être reliée pour former $—(CH_2)_3—$.

2. Un procédé selon la revendication 1, dans lequel $R^2$ est l'hydrogène ou un groupe protecteur triorganosilyle; $R^dR^eR^fSi$- dans lequel $R^d$, $R^e$ et $R^f$ sont choisis parmi les groupes alkyle ayant 1 à 6 atomes de carbone; et où $R^a$, $R^b$ et $R^c$ sont choisis indépendamment dans le groupe constitué par les groupes alkyle ayant 1 à 6 atomes de carbone, phényle et alkylphényle ayant 7 à 12 atomes de carbone.

3. Un procédé selon la revendication 1, dans lequel l'agent d'acétylation est le N-acétylimidazole.

4. Un procédé selon les revendications 2 ou 3, dans lequel $R^2$ est le triorganosilyle.

5. Un procédé pour préparer:

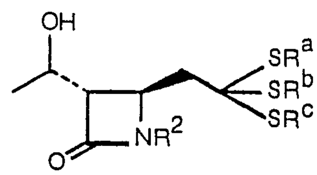

comprenant le traitement:

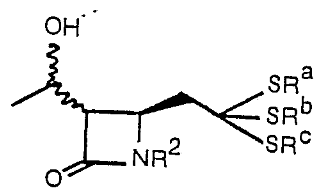

avec un agent d'oxydation pour donner:

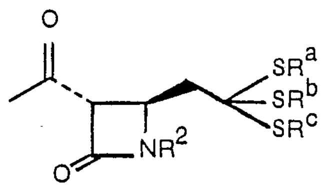

suivi du traitement avec un agent réducteur; dans lequel $R^2$, $R^a$, $R^b$ et $R^c$ sont définis de la même manière que dans la revendication 1.

6. Un procédé selon la revendication 5, dans lequel $R^2$ est l'hydrogène ou un groupe protecteur triorganosilyle: $R^dR^eR^fSi$- dans lequel $R^d$, $R^e$ et $R^f$ sont choisis indépendamment parmi les groupes alkyle ayant 1 à 6 atomes de carbone; et où $R^a$, $R^b$ et $R^c$ sont choisis indépendamment dans le groupe constitué par les groupes alkyle ayant 1 à 6 atomes de carbone, phényle et alkylphényle ayant 7 à 12 atomes de carbone.

7. Un procédé selon les revendications 5 ou 6, dans lequel $R^2$ est un triorganosilyle.

**Patentansprüche**

1. Ein Verfahren zur Herstellung von Verbindungen der Formel:

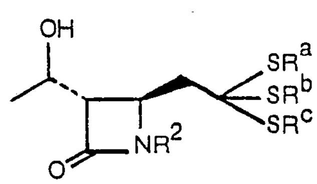

umfassend die Behandlung einer Verbindung der Formel:

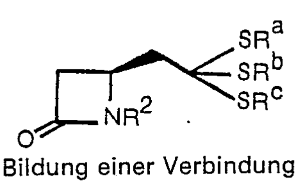

mit einem Acetylierungsmittel unter Bildung einer Verbindung der Formel:

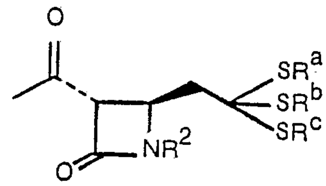

gefolgt von der Behandlung mit einem Reduktionsmittel; worin $R^2$ aus Wasserstoff oder einer Schutzgruppe ausgewählt ist; $R^a$, $R^b$ und $R^c$ unabhängig voneinander aus: Alkyl, Phenyl und Phenylalkyl ausgewählt sind, worin das Alkyl 1 bis 6 Kohlenstoffatome hat, und irgendein einzelnes Paar von $R^a$, $R^b$ oder $R^c$ unter Bildung von $—(CH_2)_3—$ verbunden sein kann.

2. Ein Verfahren nach Anspruch 1, worin $R^2$ Wasserstoff oder eine Triorganosilylschutzgruppe $R^dR^eR^fSi$- ist, worin $R^d$, $R^e$ und $R^f$ unabhängig voneinander aus Alkyl mit 1 bis 6 Kohlenstoffatomen ausgewählt sind; und worin $R^a$, $R^b$ und $R^c$ unabhängig voneinander aus der Gruppe ausgewählt sind, die aus Alkyl mit 1 bis 6 Kohlenstoffatomen, Phenyl und Alkylphenyl mit 7 bis 12 Kohlenstoffatomen besteht.

3. Ein Verfahren nach Anspruch 1, worin das Acetylierungsmittel N-Acetylimidazol ist.

4. Ein Verfahren nach Anspruch 2 oder 3, worin $R^2$ Triorganosilyl ist.

5. Ein Verfahren zur Herstellung von Verbindungen der Formel:

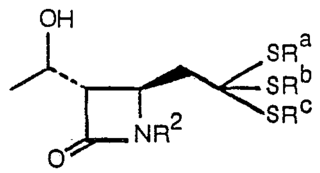

umfassend die Behandlung einer Verbindung der Formel:

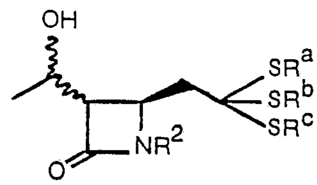

mit einem Oxydationsmittel unter Bildung einer Verbindung der Formel:

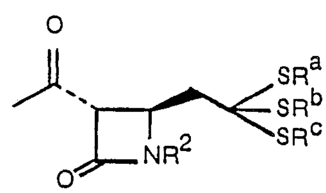

gefolgt von der Behandlung mit einem Reduktionsmittel; worin $R^2$, $R^a$, $R^b$ und $R^c$ wie im Anspruch 1 definiert sind.

6. Ein Verfahren nach Anspruch 5, worin $R^2$ Wasserstoff oder eine Triorganosilylschutzgruppe: $R^dR^eR^fSi$- ist, worin $R^d$, $R^e$ und $R^f$ unabhängig voneinander aus Alkyl mit 1 bis 6 Kohlenstoffatomen ausgewählt sind; und worin $R^a$, $R^b$ und $R^c$ unabhängig voneinander aus der Gruppe ausgewählt sind, die aus Alkyl mit 1 bis 6 Kohlenstoffatomen, Phenyl und Alkylphenyl mit 7 bis 12 Kohlenstoffatomen besteht.

7. Ein Verfahren nach Anspruch 5 oder 6, worin $R^2$ Triorganosilyl ist.